# EUROPÄISCHE PATENTSCHRIFT

(11) **EP 3 009 183 B1**
(45) Veröffentlichungstag und Bekanntmachung des Hinweises auf die Patenterteilung: **11.01.2017**
(21) Anmeldenummer: 14189052.5
(22) Anmeldetag: 15.10.2014
(51) Int. Cl.: B01F 1/00, A61M 1/16

(54) **Dialysekonzentrat-Herstellungsanordnung**
Dialysis concentrate manufacturing assembly
Système de fabrication de concentré pour dialyse

(43) Veröffentlichungstag der Anmeldung: 20.04.2016
(73) Patentinhaber: Dumschat, Christoph, 26789 Leer (DE)
(72) Erfinder: Dumschat, Christoph, 26789 Leer (DE)
(74) Vertreter: Patentanwälte ter Smitten Eberlein Rütten Partnerschaftsgesellschaft

(56) Entgegenhaltungen:
- EP-A1- 2 623 188
- DE-B3- 10 313 965

## Beschreibung

Die Erfindung bezieht sich auf eine Dialysekonzentrat-Herstellungsanordnung zur Herstellung einer Dialysekonzentratflüssigkeit durch Lösen eines Trockenkonzentrats in Wasser und auf ein Verfahren zur Herstellung der Dialysekonzentratflüssigkeit mit der Herstellungsanordnung.

Für die Durchführung einer typischen Dialysebehandlung werden üblicherweise 150-200 I Dialyseflüssigkeit benötigt. Die Dialyseflüssigkeit wird in der Regel in dem Dialysegerät aus zwei Dialysekonzentratflüssigkeiten und Wasser erzeugt, die beispielsweise in einem Verhältnis 1/35 miteinander vermischt werden. Für die Herstellung der Dialyseflüssigkeit werden eine basische Dialysekonzentratflüssigkeit, die üblicherweise aus einer Natriumhydrogencarbonat-Lösung definierter Konzentration besteht, und eine saure Dialysekonzentratflüssigkeit, die alle übrigen für die Dialyseflüssigkeit erforderlichen Bestandteile in der erforderlichen Konzentration enthält, mit Wasser homogen vermischt.

Für die Durchführung einer typischen Dialysebehandlung wird bis zu 5 l saure Dialysekonzentratflüssigkeit benötigt, wobei ca. 90% hiervon wiederum Wasser ist. Aus DE 103 13 965 B3 ist eine Dialysekonzentrat-Herstellungsanordnung bekannt, durch die zur Herstellung der Dialysekonzentratflüssigkeit ein Trockenkonzentrat mit Wasser homogen vermischt wird. Das Trockenkonzentrat wird in einem mobilen und mehrfach verwendbaren Wechselbehälter geliefert, der zwei Anschlüsse zum Einleiten bzw. Ableiten von Flüssigkeit aufweist. Die beiden Anschlüsse werden am Einsatzort an eine stationäre Herstellungsanlage angeschlossen, die unter anderem einen sogenannten Vorlagebehälter, eine Förderpumpe stromabwärts eines Auslasses des Vorlagebehälters, zwei Wasserstrahlpumpen und mehrere Ventile aufweist.

Der Prozess zur Herstellung der Dialysekonzentratflüssigkeit beginnt damit, dass eine exakt bemessene Wassermenge in den Vorlagebehälter eingeleitet wird. Anschließend wird durch entsprechendes Schalten von Sperrventilen das Wasser aus dem Vorlagebehälter in den Wechselbehälter gepumpt, wodurch das Trockenkonzentrat in dem Wasser gelöst wird. Sobald der geschlossene Wechselbehälter vollständig mit Flüssigkeit gefüllt ist, fließt diese durch einen Ablauf am oberen Ende des Wechselbehälters zurück zu dem Vorlagebehälter. Die Flüssigkeit wird so lange zwischen dem Vorlagebehälter und dem Wechselbehälter und wieder zurück im Kreis gepumpt, bis das Trockenkonzentrat vollständig und homogen im gesamten Flüssigkeitsvolumen gelöst ist.

Die Flüssigkeit wird unter Druck in den Wechselbehälter hineingepumpt und fließt unter Druck aus dem Auslass heraus zu dem Vorlagebehälter. Der Wechselbehälter ist also kontinuierlich einem Überdruck ausgesetzt und darf keine Undichtigkeiten aufweisen, da andernfalls nicht unerhebliche Flüssigkeitsmengen verloren gehen können, ohne dass dies ohne weiteres bemerkt werden kann. Hierdurch kann sich auch die Konzentration der chemischen Bestandteile des Trockenkonzentrats in der Gesamtflüssigkeit in unerwünschter Weise verändern.

Aufgabe der Erfindung vor diesem Hintergrund ist es, eine Dialysekonzentrat-Herstellungsanordnung bzw. ein Verfahren zur Herstellung einer Dialysekonzentratflüssigkeit zu schaffen, bei der bzw. bei dem die **Flüssigkeitsverluste** reduziert sind.

Diese Aufgabe wird erfindungsgemäß gelöst mit einer Dialysekonzentrat-Herstellungsanordnung mit den Merkmalen des Anspruchs 1 und einem Verfahren zur Herstellung einer Dialysekonzentratflüssigkeit mit den Merkmalen des Anspruchs 9.

Die erfindungsgemäße Dialysekonzentrat-Herstellungsanordnung weist ebenfalls einen mobilen Wechselbehälter mit dem Trockenkonzentrat auf. Der Wechselbehälter weist einen Ablaufanschluss, durch den die Flüssigkeit den Wechselbehälter während des Mischprozesses verlässt, und einen Kombianschluss auf, der während des Mischprozesses sowohl zum Befüllen als auch zum Entleeren des Wechselbehälters genutzt wird. Der Wechselbehälter ist weitgehend gas-und flüssigkeitsdicht, so dass er ohne Flüssigkeitsverlust jedenfalls mit einem geringen Überdruck beaufschlagt werden kann.

Die stationäre Herstellungsanlage weist einen Vorlagebehälter mit einem Auslass auf, der bevorzugt am Boden des Vorlagebehälters angeordnet ist, und durch den die Flüssigkeit aus dem Vorlagebehälter abläuft. Der Vorlagebehälter weist ferner einen Mischeinlass auf, der bevorzugt oben in den Vorlagebehälter mündet. Die Herstellungsanlage weist ferner eine Förderpumpe stromabwärts hinter dem Vorlagebehälter-Auslass auf. Die Förderpumpe ist bevorzugt als elektrische Verdrängerpumpe ausgebildet. Weiter stromabwärts hinter der Förderpumpe ist eine erste Wasserstrahlpumpe angeordnet. Die erste Wasserstrahlpumpe weist einen Treibeinlass auf, durch den die von der Förderpumpe kommende Flüssigkeit in die Wasserstrahlpumpe unter Druck einströmt. Die Wasserstrahlpumpe weist einen Sauganschluss auf, der über eine entsprechende Verbindungsleitung fluidisch mit dem Kombianschluss des Wechselbehälters verbunden ist.

Ferner ist eine zweite Wasserstrahlpumpe stromabwärts hinter der Förderpumpe vorgesehen, die fluidisch parallel zu der ersten Wasserstrahlpumpe angeordnet ist. Der Treibeinlass der zweiten Wasserstrahlpumpe wird durch die von der Förderpumpe geförderte Flüssigkeit unter Druck gespeist. Der Sauganschluss der zweiten Wasserstrahlpumpe ist fluidisch mit dem Ablaufanschluss des Wechselbehälters verbunden. Der Auslass der zweiten Wasserstrahlpumpe ist fluidisch mit dem Vorlagebehälter-Mischeinlass verbunden, so dass die von dem Auslass der zweiten Wasserstrahlpumpe kommende Flüssigkeit in den Vorlagebehälter eingeleitet wird.

Die Förderpumpe speist also die Treibeinlässe der beiden Wasserstrahlpumpen, deren Sauganschlüsse mit dem Kombianschluss und dem Ablaufanschluss des Wechselbehälters fluidisch verbunden sind.

Sobald der Vorlagebehälter mit der definierten Wassermenge gefüllt ist, kann das Mischen der Dialysekonzentratflüssigkeit aus dem Trockenkonzentrat in dem angeschlossenen Wechselbehälter und der definierten Wassermenge in dem Vorlagebehälter beginnen. Hierzu werden die gegebenenfalls vorhandenen Sperrventile so geschaltet, dass der Strömungspfad von der Förderpumpe zu den Treibeinlässen der beiden Wasserstrahlpumpen geöffnet ist, jedoch ein Sperrventil hinter dem Auslass der ersten Wasserstrahlpumpe geschlossen ist.

Sobald die Förderpumpe in Betrieb ist, pumpt diese das aus dem Vorlagebehälter-Auslass ausfließende Wasser in etwa gleicher Flussrate und unter etwa gleichem Druck zu den beiden Wasserstrahlpumpen. In der ersten Wasserstrahlpumpe strömt das Wasser in Gegenrichtung durch den Sauganschluss und von dort durch den Kombianschluss in den Wechselbehälter, wo das Trockenkonzentrat in dem einströmenden Wasser gelöst wird. Gleichzeitig arbeitet die zweite Wasserstrahlpumpe als Vakuumpumpe und evakuiert den Wechselbehälter auf diese Weise, bis der Flüssigkeitspegel in dem Wechselbehälter den Ablaufanschluss erreicht. Sobald dies geschehen ist, arbeitet die zweite Wasserstrahlpumpe als saugende Flüssigkeitspumpe, und saugt die Flüssigkeit aus dem Wechselbehälter ab, die von dem Auslass der zweiten Wasserstrahlpumpe aus wieder zurück in den Vorlagebehälter fließt.

Die Flüssigkeit wird auf diese Weise zwischen dem Vorlagebehälter und dem Wechselbehälter ständig im Kreis gepumpt. Die Flüssigkeit wird mit einem gewissen Überdruck durch die erste Wasserstrahlpumpe in den Wechselbehälter hineingepumpt und mit einem gewissen Unterdruck durch die zweite Wasserstrahlpumpe aus dem Wechselbehälter abgesaugt. Wenn der Gegendruck in dem Kombianschluss steigt, verringert sich die Flussrate in der ersten Wasserstrahlpumpe, so dass sich die Flussrate in der zweiten Wasserstrahlpumpe entsprechend erhöht, so dass sich auch der von der zweiten Wasserstrahlpumpe generierte Unterdruck an ihren Sauganschluss erhöht. Die beiden Wasserstrahlpumpen regeln sich also gegenseitig.

Auf diese Weise stellt sich innerhalb des fluiddichten Wechselbehälters ein statischer Druck ein, der nur wenig unter- oder oberhalb des atmosphärischen Umgebungsdruckes liegt. Versuche haben ergeben, dass die Druckdifferenz zwischen dem Umgebungsdruck und dem Wechselbehälter-Innendruck lediglich in der Größenordnung von 0,1 bar liegt.

Durch die geringe Druckdifferenz wird eine praktisch drucklose Mischphase realisiert, so dass selbst bei Undichtigkeiten des mobilen Wechselbehälters die hierdurch verursachten Leckverluste sehr gering ausfallen. Dies ist auch deshalb von großer Bedeutung, weil an die Einhaltung des exakten Mischungsverhältnisses des Trockenkonzentrats in dem Wasser sehr hohe Anforderungen gestellt werden müssen. Da der Wechselbehälter keinem nennenswerten Innen-Überdruck ausgesetzt ist, wird dieser insbesondere im Bereich der Dichtungen und Durchführungen weniger gestresst, so dass hierdurch die Haltbarkeit des Wechselbehälters verbessert wird.

Vorzugsweise ist der Auslass der ersten Wasserstrahlpumpe fluidisch mit einem Fülleinlass des Vorlagebehälters verbunden. Fluidisch zwischen dem Auslass der ersten Wasserstrahlpumpe und dem Vorlagebehälter-Fülleinlass ist ein erstes Sperrventil angeordnet, so dass bei geschlossenen erstem Sperrventil die von der Förderpumpe geförderte Flüssigkeit im Gegenstrom durch den Sauganschluss der ersten Wasserstrahlpumpe in den Wechselbehälter gepumpt wird. Bei geöffnetem ersten Sperrventil dagegen wird die Flüssigkeit durch den Sauganschluss der ersten Wasserstrahlpumpe aus dem Wechselbehälter-Kombianschluss abgesaugt. Durch Umschalten des ersten Sperrventils wird die Strömungsrichtung des Sauganschlusses der ersten Wasserstrahlpumpe umgeschaltet.

Nachdem in der Mischphase das Trockenkonzentrat vollständig und homogen in dem gesamten Wasservolumen gelöst ist, wird das zuvor geschlossene erste Sperrventil geöffnet, so dass in der anschließenden Sammelphase die gesamte Systemflüssigkeit einschließlich der Flüssigkeit in dem Wechselbehälter schließlich vollständig in den Vorlagebehälter gepumpt wird. Sobald dies vollendet ist, ist der gesamte Mischprozess abgeschlossen, so dass der Wechselbehälter abgeklemmt und entfernt werden kann.

Besonders bevorzugt ist zwischen dem Treibeinlass der zweiten Wasserstrahlpumpe und der Förderpumpe ein zweites Sperrventil angeordnet. Während der Mischphase wird bei geöffnetem zweiten Sperrventil durch den Sauganschluss Fluid, also Gas oder Flüssigkeit, von dem Wechselbehälter-Ablaufanschluss angesaugt. Während der anschließenden Sammelphase wird das zweite Sperrventil geschlossen, damit die zweite Wasserstrahlpumpe keinen Unterdruck mehr in dem Wechselbehälter generiert. Auf diese Weise wird die zweite Wasserstrahlpumpe während der Sammelphase fluidisch stillgelegt.

Gemäß einer bevorzugten Ausgestaltung mündet der Kombianschluss des Wechselbehälters in eine Fluidöffnung am tiefstgelegenen des Wechselbehälters. Auf diese Weise wird sichergestellt, dass in der Sammelphase der Wechselbehälter restlos geleert wird, so dass keine Flüssigkeit in dem Wechselbehälter verbleibt. Dies ist deshalb von großer Wichtigkeit, weil andernfalls die exakte Einhaltung des vorgegebenen Mischungsverhältnisses des Trockenkonzentrats in dem Wasser nicht sichergestellt ist.

Vorzugsweise mündet der Wechselbehälter-Ablaufanschluss in einer Fluidöffnung im oberen Bereich des Wechselbehälters, und besonders bevorzugt an der höchsten Stelle innerhalb des Wechselbehälters. Auf diese Weise wird eine maximale Strömungsweglänge der Flüssigkeit innerhalb des Wechselbehälters sichergestellt, wodurch eine bestmögliche Homogenität des gelösten Trockenkonzentrats in der Flüssigkeit befördert wird.

Gemäß einer bevorzugten Ausgestaltung sind die beiden Wasserstrahlpumpen technisch identisch ausgebildet. Hinter der Förderpumpe ist bevorzugt eine fluidische Verzweigung vorgesehen, von wo aus jeder Arm der Verzweigung zu dem jeweiligen Treibeinlass der beiden Wasserstrahlpumpen führt. Die beiden Wasserstrahlpumpen werden auf diese Weise von der Förderpumpe symmetrisch bedient, also mit derselben Flussrate beaufschlagt. Wegen der fluidischen und der baulichen Symmetrie der beiden Wasserstrahlpumpen stabilisiert sich ihre jeweilige Pumpleistung auf ungefähr gleichem Niveau. Hierdurch wird Insbesondere während der Mischphase sichergestellt, dass der Innendruck in dem Wechselbehälter nur gering von dem atmosphärischen Umgebungsdruck abweicht.

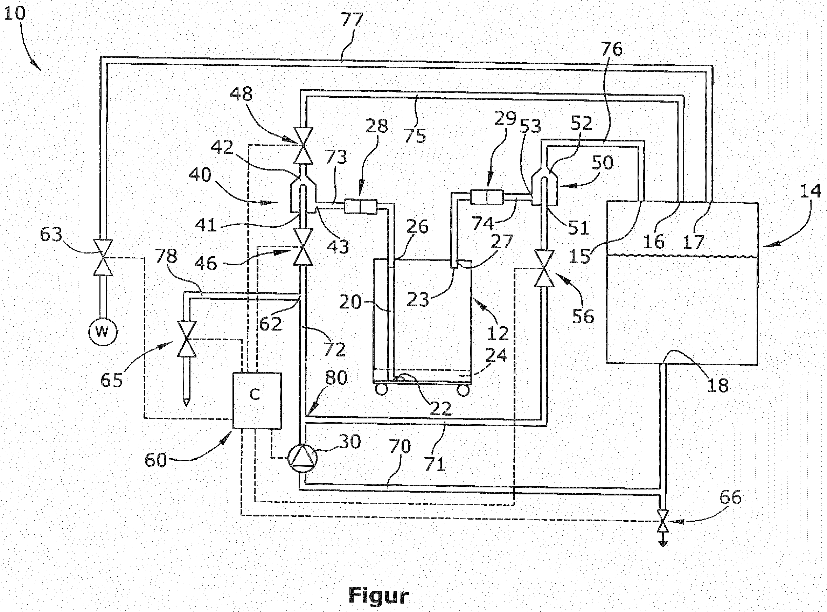

Im Folgenden wird ein Ausführungsbeispiel der Erfindung anhand der Zeichnung näher erläutert.

Die Figur zeigt schematisch eine Dialysekonzentrat-Herstellungsanordnung 10 zur Herstellung einer Dialysekonzentratflüssigkeit durch Lösen eines Trockenkonzentrats in Wasser. Die Herstellungsanordnung 10 besteht aus einem mobilen Wechselbehälter 12 mit einem Trockenkonzentrat 24 und aus einer stationären Herstellungsanlage, die eine Vielzahl von Komponenten aufweist.

Der mobile Wechselbehälter 12 ist tonnenartig ausgebildet und fluidisch geschlossen. Der Wechselbehälter 12 kann Räder für den Transport aufweisen. Der Wechselbehälter 12 weist oben einen Ablaufanschluss 27 auf, dessen zugeordnete innenseitige Öffnung 23 am obersten Punkt innerhalb des Wechselbehälters 12 vorgesehen ist. Der Wechselbehälter 12 weist oben ferner einen Kombianschluss 26 auf, der innerhalb des Wechselbehälters 12 in ein senkrechtes Steigrohr 20 mündet, an dessen unterem Ende eine in tangentialer Strömungsrichtung öffnende Öffnung 22 vorgesehen ist. Dem Kombianschluss 26 und dem Ablaufanschluss 27 ist fluidisch jeweils eine Anschlusskupplung 28,29 zugeordnet, über die der mobile Wechselbehälter 12 auf einfache Weise fluidisch mit der Herstellungsanlage verbunden wird.

Die Herstellungsanlage weist einen sogenannten Vorlagebehälter 14 auf, der oben einen Mischeinlass 15, einen Sammeleinlass 16 und einen Fülleinlass 17 aufweist. An seinem tiefsten Punkt weist der Vorlagebehälter 14 einen Auslass 18 auf. Der Vorlagebehälter-Auslass 18 ist über eine Auslassleitung 70 mit einer Verdränger-Förderpumpe 30 fluidisch verbunden, die die Flüssigkeit aus dem Vorlagebehälter 14 fördert. Flussabwärts hinter der Förderpumpe 30 ist eine fluidische Verzweigung 80 vorgesehen, wobei die eine Zweigleitung 72 über ein drittes Sperrventil 46 zu einer ersten Wasserstrahlpumpe 40 führt und die andere Zweigleitung 71 über ein zweites Sperrventil 56 zu einer zweiten Wasserstrahlpumpe 50 führt, die technisch identisch zu der ersten Wasserstrahlpumpe 40 ist.

Jede der beiden Wasserstrahlpumpen 40,50 weist jeweils einen Treibeinlass 41,51, einen Auslass 42,52 und einen Sauganschluss 43,53 auf. Der Sauganschluss 43 der ersten Wasserstrahlpumpe 40 ist über eine Leitung 73 mit dem Wechselbehälter-Kombianschluss 26 bzw. der dem Kombianschluss 26 zugeordneten Kupplung 28 fluidisch verbunden. Der Sauganschluss 53 der zweiten Wasserstrahlpumpe 50 ist mit dem Wechselbehälter-Ablaufanschluss 27 bzw. der dem Ablaufanschluss 27 zugeordneten Kupplung 29 über eine Leitung 74 fluidisch verbunden. Der Auslass 52 der zweiten Wasserstrahlpumpe 50 ist über eine Leitung 76 mit dem Mischeinlass 15 des Vorlagebehälters 14 fluidisch verbunden. Der Auslass 42 der ersten Wasserstrahlpumpe 40 ist über ein zwischengeschaltetes erstes Sperrventil 48 und über eine Leitung 75 mit dem Sammeleinlass 16 des Vorlagebehälters 14 fluidisch verbunden.

Von einer Wasserquelle W fließt bei Bedarf Wasser, beispielsweise aufbereitetes und filtriertes Leitungswasser, über ein viertes Sperrventil 63 und eine Füllleitung 77 zu dem Fülleinlass 17 des Vorlagebehälters 14.

Zwischen dem Vorlagebehälter-Auslass 18 und der Förderpumpe 30 ist ein Spülabzweig mit einem Ablassventil 66 in dem Nebenzweig vorgesehen, über den der Vorlagebehälter 14 vollständig in einen Abfluss entleert werden kann.

Zwischen der Förderpumpe 30 und dem dritten Sperrventil 46 ist ein weiterer fluider Abzweig 62 vorgesehen, von dem eine Förderleitung 78 mit einem fünften Sperrventil 65 zum Abpumpen der fertig gemischten Dialysekonzentratflüssigkeit abzweigt.

Die dargestellten Sperrventile, die Förderpumpe 30 und mehrere nicht dargestellte Sensoren, beispielsweise Flusssensoren, Dichtesensoren etc. werden von einer Anlagensteuerung 60 gelesen, gesteuert und kontrolliert, die den gesamten Herstellungsprozess steuert.

Der Herstellungsprozess läuft wie folgt ab:
Zunächst wird der neu mit dem Trockenkonzentrat 24 befüllte Wechselbehälter 12 über die beiden Kupplungen 28,29 fluidisch mit der Herstellungsanlage verbunden. Nachdem anschließend der Start des Herstellungsprozesses ausgelöst ist, öffnet die Anlagensteuerung 60 das fünfte Sperrventil 63, so dass Wasser von der Wasserquelle W über die Füllleitung 77 und den Fülleinlass 17 in den Vorlagebehälter 14 fließt. Während der Füllphase ist die Förderpumpe 30 nicht in Betrieb und sind alle übrigen Sperrventile geschlossen. Sobald der Vorlagebehälter 14 mit der definierten Wassermenge gefüllt ist, wird das fünfte Sperrventil 63 geschlossen, so dass kein Wasser mehr nachströmt

An die Füllphase schließt sich die Mischphase an. Hierzu öffnet die Anlagensteuerung 60 das dritte Sperrventil 46 und das zweite Sperrventil 56, und aktiviert die Förderpumpe 30. Das erste Sperrventil 48 bleibt geschlossen. Die erste Wasserstrahlpumpe 40 wird auf diese Weise im Gegenstrom und die zweite Wasserstrahlpumpe 50 im Saugmodus betrieben. Die Förderpumpe 30 fördert das Wasser aus dem Vorlagebehälter zu den beiden Wasserstrahlpumpen 40,50. Da das erste Sperrventil 48 geschlossen ist, strömt das Wasser durch den Sauganschluss 43 der ersten Wasserstrahlpumpe 40 im Gegenstrom in den Mischeinlass 26 des Wechselbehälters 12, so dass der Flüssigkeitspegel in dem Wechselbehälter 12 steigt. Das Wasser strömt tangential aus der Steigrohr-Öffnung 22 aus, so dass hierdurch das pulverartige Trockenkonzentrat 22 von dem Wasserstrom mitgerissen und in dem Wasser gelöst wird. Gleichzeitig erzeugt die zweite Wasserstrahlpumpe 50, die in dieser Phase zunächst als Vakuumpumpe arbeitet, einen gewissen Unterdruck, so dass der Innenraum des Wechselbehälters 12 im Verhältnis zum Umgebungsdruck nahezu drucklos ist.

Sobald der Flüssigkeitspegel in dem Wechselbehälter 12 den Behälterdeckel erreicht, fließt die Flüssigkeit durch den Ablauf 27 und die Leitungen 74,76 in den Mischeinlass 15 des Vorlagebehälters 14. In dieser Phase arbeitet die zweite Wasserstrahlpumpe 50 als Flüssigkeits-Saugpumpe, so dass der Innenraum des Wechselbehälters 12 im Verhältnis zum atmosphärischen Umgebungsdruck nahezu drucklos ist. Das gesamte im System befindliche Flüssigkeitsvolumen ist größer als das Füllvolumen des Wechselbehälters 12, so dass die Flüssigkeit zwischen dem Vorlagebehälter 14 und dem Wechselbehälter 12 ständig im Kreis gepumpt wird. Hierdurch wird eine Homogenisierung aller Bestandteile des Trockenkonzentrats in der Flüssigkeit erreicht, bis die Homogenität schließlich so hoch ist, dass die Flüssigkeit als Dialysekonzentratflüssigkeit genutzt werden kann. Die Homogenität der Flüssigkeit wird beispielsweise über Dichtesensoren und/oder auf elektrochemische oder auf optische Weise ermittelt.

Sobald die Mischphase beendet ist, wird das zweite Sperrventil 56 geschlossen und das erste Sperrventil 48 geöffnet. Die erste Wasserstrahlpumpe 40 wird dann im Saugmodus betrieben und die zweite Wasserstrahlpumpe 50 ist nicht in Betrieb, so dass über den Sauganschluss 43 der ersten Wasserstrahlpumpe 40 der Wechselbehälter 12 vollständig leergepumpt und die abgepumpte Flüssigkeit über die Leitung 75 zu dem Sammeleinlass 16 des Vorlagebehälters 14 gepumpt wird. Sobald der Wechselbehälter 12 auf diese Weise vollständig leergepumpt ist, wird die Sammelphase beendet, indem das dritte Sperrventil 46 geschlossen wird. In dem Vorlagebehälter 14 befindet sich nun die gesamte Menge an auf diese Weise hergestellter Dialysekonzentratflüssigkeit.

Schließlich wird die Dialysekonzentratflüssigkeit aus dem Vorlagebehälter 14 abgepumpt, indem das vierte Sperrventil 65 geöffnet wird, so dass die Dialysekonzentratflüssigkeit über die Förderleitung 78 in einen entsprechenden Transportbehälter abgepumpt werden kann.

## Patentansprüche

1. Dialysekonzentrat-Herstellungsanordnung (10) zur Herstellung einer Dialysekonzentratflüssigkeit durch Lösen eines Trockenkonzentrats (24) in Wasser, mit
einem mobilen Wechselbehälter (12) mit dem Trockenkonzentrat (24), wobei der Wechselbehälter (12) einen Ablaufanschluss (27) und einen Kombianschluss (26) aufweist, und
einer stationären Herstellungsanlage, die aufweist:
einen Vorlagebehälter (14) mit einem Mischeinlass (15) und einem Auslass (18),
einer Förderpumpe (30) stromabwärts hinter dem Vorlagebehälter-Auslass (18),
einer ersten Wasserstrahlpumpe (40) stromabwärts hinter der Förderpumpe (30), wobei die Wasserstrahlpumpe (40) einen Treibeinlass (41), einen Sauganschluss (43) und einen Auslass (42) aufweist, und wobei der Treibeinlass (41) fluidisch mit der Förderpumpe (30) und der Sauganschluss (43) fluidisch mit dem Wechselbehälter-Kombianschluss (26) verbunden ist,
und
einer zweiten Wasserstrahlpumpe (50) stromabwärts hinter der Förderpumpe (30) und parallel zu der ersten Wasserstrahlpumpe (40), wobei die zweite Wasserstrahlpumpe (50) einen Treibeinlass (51), einen Sauganschluss (53) und einen Auslass (52) aufweist, und wobei der Treibeinlass (51) fluidisch mit der Förderpumpe (30), der Sauganschluss (53) fluidisch mit dem Wechselbehälter-Ablaufanschluss (27) und der Auslass (52) fluidisch mit dem Vorlagebehälter-Mischeinlass (15) verbunden ist.

2. Dialysekonzentrat-Herstellungsanordnung (10) nach Anspruch 1, wobei der Auslass (42) der ersten Wasserstrahlpumpe (40) fluidisch mit einem Fülleinlass (16) des Vorlagebehälters (14) verbunden ist und fluidisch zwischen dem Auslass (42) der ersten Wasserstrahlpumpe (40) und dem Vorlagebehälter-Fülleinlass (16) ein erstes Sperrventil (48) angeordnet ist, so dass bei geöffnetem ersten Sperrventil (48) durch den Sauganschluss (43) der ersten Wasserstrahlpumpe (40) Fluid aus dem Wechselbehälter-Kombianschluss (26) abgesaugt wird und bei geschlossenem ersten Sperrventil (48) durch den Sauganschluss (43) der ersten Wasserstrahlpumpe (40) Flüssigkeit in den Wechselbehälter-Kombianschluss (26) gepumpt wird.

3. Dialysekonzentrat-Herstellungsanordnung (10) nach einem der vorangegangenen Ansprüche, wobei zwischen dem Treibeinlass (51) der zweiten Wasserstrahlpumpe (50) und der Förderpumpe (30) ein zweites Sperrventil (56) angeordnet ist, so dass bei geöffnetem zweiten Sperrventil (56) durch den Sauganschluss (53) der zweiten Wasserstrahlpumpe (50) Fluid von dem Wechselbehälter-Ablaufanschluss (27) angesaugt wird.

4. Dialysekonzentrat-Herstellungsanordnung (10) nach einem der vorangegangenen Ansprüche, wobei der Kombianschluss (26) in einer Fluidöffnung (22) am tiefstgelegenen Punkt des Wechselbehälters (12) mündet.

5. Dialysekonzentrat-Herstellungsanordnung (10) nach einem der vorangegangenen Ansprüche, wobei der Wechselbehälter-Ablaufanschluss (27) in einer Fluidöffnung (23) im oberen Bereich des Wechselbehälters (12) mündet.

6. Dialysekonzentrat-Herstellungsanordnung (10) nach einem der vorangegangenen Ansprüche, wobei die beiden Wasserstrahlpumpen (40,50) identisch ausgebildet sind.

7. Dialysekonzentrat-Herstellungsanordnung (10) nach einem der vorangegangenen Ansprüche, wobei die Förderpumpe (30) als Verdrängerpumpe ausgebildet ist.

8. Dialysekonzentrat-Herstellungsanordnung (10) nach einem der vorangegangenen Ansprüche, wobei fluidisch zwischen der Förderpumpe (30) und der ersten Wasserstrahlpumpe (40) eine Dialysekonzentratflüssigkeits- Förderleitung (78) abzweigt, und zwischen dem Förderleitungs-Abzweig und der ersten Wasserstrahlpumpe (40) ein drittes Sperrventil (46) angeordnet ist, so dass bei geschlossenem dritten Sperrventil (46) die Förderpumpe (30) Dialysekonzentratflüssigkeit aus dem Vorlagebehälter (14) in die Dialysekonzentratflüssigkeits-Förderleitung (78) pumpt.

9. Verfahren zur Herstellung einer Dialysekonzentratflüssigkeit mit einer Dialysekonzentrat-Herstellungsanordnung (10) nach Ansprüchen 1, 2 und 3, und weiter optional nach einem der Ansprüche 4 bis 8, mit den Verfahrensschritten zum Mischen der Dialysekonzentratflüssigkeit aus dem Trockenkonzentrat (22) und Wasser:
Füllen des Vorlagebehälters (14) mit Wasser,
Schließen des ersten Sperrventils (48),
Öffnen des zweiten Sperrventils (56), und
Einschalten der Förderpumpe (30).

10. Verfahren zur Herstellung einer Dialysekonzentratflüssigkeit nach Anspruch 9, mit den Verfahrensschritten:
nach dem Mischen: Schließen des zweiten Sperrventils (56) und Öffnen des ersten Sperrventils (48).

## Claims

1. A dialysis concentrate production system (10) for producing a dialysis concentrate liquid by dissolving a dry concentrate (24) in water comprising
a mobile interchangeable container (12) holding the dry concentrate (24), wherein the interchangeable container (12) has a discharge port (27) and a combined port (26), and
a stationary production system comprising:
a storage container (14) with a mixing inlet (15) and an outlet (18),
a delivery pump (30) downstream of the storage container outlet (18),
a first water jet pump (40) downstream of the delivery pump (30), wherein the water jet pump (40) has a driving inlet (41), a suction port (43) and an outlet (42), and wherein the driving inlet (41) is in fluid communication with the delivery pump (30) and the suction port (43) is in fluid communication with the combined port (26) interchangeable container, and
a second water jet ump (50) downstream of the delivery pump (30) and parallel to the first water jet pump (40), wherein the second water jet pump (50) has a driving inlet (51), a suction port (53) and an outlet (52), and wherein the driving inlet (51) is in fluid communication with the delivery pump (30), the suction port (53) is in fluid communication with the interchangeable container discharge port (27) and the outlet (52) is in fluid communication with the storage container mixing inlet (15).

2. The dialysis concentrate production system (10) of claim 1, wherein the outlet (42) of the first water jet pump (40) is in fluid communication with a filling inlet (16) of the storage container (14), and wherein a first shut-off valve (48) is arranged fluidically between the outlet (42) of the first water jet pump (40) and the storage container filling inlet (16) so that, with the first shut-off valve (48) open, fluid is drawn from the combined port (26) of the interchangeable container via the suction port (43) of the first water jet pump (40) and, with the first shut-off valve (48) closed, liquid is pumped into the combined port (26) of the interchangeable container via the suction port (43) of the first water jet pump (40).

3. The dialysis concentrate production system (10) of one of the preceding claims, wherein a second shut-off valve (56) is arranged between the driving inlet (51) of the second water jet pump (50) and the delivery pump (30) so that, with the second shut-off valve (56) open, fluid is drawn from the interchangeable container discharge port (27) through the suction port (53) of the second water jet pump (50).

4. The dialysis concentrate production system (10) of one of the preceding claims, wherein the combined port (26) terminates in a fluid opening (22) at the lowest point of the interchangeable container (12).

5. The dialysis concentrate production system (10) of one of the preceding claims, wherein the interchangeable container discharge port (27) terminates in a fluid opening (23) in the top portion of the interchangeable container (12).

6. The dialysis concentrate production system (10) of one of the preceding claims, wherein the two water jet pumps (40, 50) are identical in structure.

7. The dialysis concentrate production system (10) of one of the preceding claims, wherein the delivery pump (30) is designed as a displacement pump.

8. The dialysis concentrate production system (10) of one of the preceding claims, wherein a dialysis concentrate liquid delivery conduit (78) branches fluidically between the delivery pump (30) and the first water jet pump (40), and wherein a third shut-off valve (46) is arranged between the delivery conduit branch and the first water jet pump (40), so that, with the third shut-off valve (46) closed, the delivery pump (30) pumps dialysis concentrate liquid from the storage container (14) into the dialysis concentrate liquid delivery conduit (78).

9. A method for producing a dialysis concentrate liquid with a dialysis concentrate production system (10) of one of claims 1, 2 and 3, and optionally one of claims 4 to 8, comprising the following method steps for mixing the dialysis concentrate liquid from the dry concentrate (24) and water:
filling the storage container (14) with water,
closing the first shut-off valve (48),
opening the second shut-off valve (56) and
activating the delivery pump (30).

10. The method of producing a dialysis concentrate liquid of claim 9, with the method steps of:
after the mixing:
closing the second shut-off valve (56) and opening the first shut-off valve (48).

## Revendications

1. Système (10) de fabrication de concentré pour dialyse destiné à la fabrication d'un liquide de concentré pour dialyse par dissolution d'un concentré sec (24) dans l'eau, avec
un conteneur (12) interchangeable mobil contenant le concentré sec (24), ledit un conteneur (12) interchangeable a un raccord de sortie (27) et un raccord combiné (26), et
un système de fabrication stationnaire comprenant:
un conteneur de stockage (14) avec une entrée de mélange (15) et une sortie (18),
une pompe d'alimentation (30) en aval de la sortie (18) du conteneur de stockage,
une première pompe à jet d'eau (40) en aval de la pompe d'alimentation (30), ladite pompe à jet d'eau (40) ayant une entrée d'entrainement (41), un raccord d'aspiration (43) et une sortie (42), et l'entrée d'entrainement (41) étant en communication fluidique avec la pompe d'alimentation (30) et le raccord d'aspiration (43) étant en communication fluidique avec le raccord combiné (26) du conteneur de stockage, et
une deuxième pompe à jet d'eau (50) en aval de la pompe d'alimentation (30) et en parallèle avec la première pompe à jet d'eau (40), ladite deuxième pompe à jet d'eau (50) ayant une entrée d'entrainement (51), un raccord d'aspiration (53) et une sortie (52), et l'entrée d'entrainement (51) étant en communication fluidique avec la pompe d'alimentation (30), le raccord d'aspiration (53) étant en communication fluidique avec le raccord de sortie (27) du conteneur interchangeable, et la sortie (52) étant en communication fluidique avec l'entrée de mélange (15) du conteneur de stockage.

2. Système (10) de fabrication de concentré pour dialyse selon la revendication 1, dans lequel la sortie (42) de la première pompe à jet d'eau (40) est en communication fluidique avec une entrée de remplissage (16) du conteneur de stockage (14) et une première soupape d'arrêt (48) est disposée fluidiquement entre la sortie (42) de la première pompe à jet d'eau (40) et l'entrée de remplissage (16) du conteneur de stockage, de sorte qu'à l'état ouvert de ladite première soupape d'arrêt (48) de la fluide est aspirée du raccord combiné (26) du conteneur interchangeable par le raccord d'aspiration (43) de la première pompe à jet d'eau (40) et, à l'état fermé de ladite première soupape d'arrêt (48) du liquide est pompé dans le raccord combiné (26) du conteneur interchangeable par le raccord d'aspiration (43) de la première pompe à jet d'eau (40).

3. Système (10) de fabrication de concentré pour dialyse selon l'une quelconque des revendications précédentes, dans lequel une deuxième soupape d'arrêt (56) es disposée entre l'entrée d'entrainement (51) de la deuxième pompe à jet d'eau (50) et la pompe d'alimentation (30), de sorte qu'à l'état ouvert de ladite deuxième soupape d'arrêt (56) de la fluide est aspirée du raccord de sortie (27) du conteneur interchangeable par le raccord d'aspiration (53) de la deuxième pompe à jet d'eau (50).

4. Système (10) de fabrication de concentré pour dialyse selon l'une quelconque des revendications précédentes, dans lequel le raccord combiné (26) s'ouvre dans une ouverture de fluide (22) au point le plus bas du conteneur interchangeable (12).

5. Système (10) de fabrication de concentré pour dialyse selon l'une quelconque des revendications précédentes, dans lequel le raccord de sortie (27) du conteneur interchangeable s'ouvre dans une ouverture de fluide (22) dans la partie haute du conteneur interchangeable (12).

6. Système (10) de fabrication de concentré pour dialyse selon l'une quelconque des revendications précédentes, dans lequel les deux pompes à jet d'eau (40, 50) sont de configuration identique.

7. Système (10) de fabrication de concentré pour dialyse selon l'une quelconque des revendications précédentes, dans lequel la pompe d'alimentation (30) est configurée comme pompe volumétrique.

8. Système (10) de fabrication de concentré pour dialyse selon l'une quelconque des revendications précédentes, dans lequel un conduit de transport (78) de liquide de concentré pour dialyse est branché fluidiquement entre la pompe d'alimentation (30) et la première pompe à jet d'eau (40), et une troisième soupape d'arrêt (46) est disposée le branchement du conduit de transport et la première pompe à jet d'eau (40), de sorte qu'à l'état fermé de ladite troisième soupape d'arrêt (46), la pompe d'alimentation (30) pompe de liquide de concentré pour dialyse du conteneur de stockage (14) dans le conduit de transport (78) de liquide de concentré pour dialyse.

9. Procédé de fabrication d'un liquide de concentré pour dialyse avec un système (10) de fabrication de concentré pour dialyse selon les revendications 1, 2 et 3 et optionellement une des revendications 4 à 8, avec les étapes de procédé pour mélanger le liquide de concentré pour dialyse à partir du concentré (22) sec et de l'eau:
remplir le conteneur de stockage (14) avec de l'eau,
fermer la première soupape d'arrêt (48),
ouvrir la deuxième soupape d'arrêt (56), et
mettre en marche la pompe d'alimentation (30).

10. Procédé de fabrication d'un liquide de concentré pour dialyse selon la revendication 9, avec les étapes de procédé:
après mélange: fermer la deuxième soupape d'arrêt (56) et ouvrir la première soupape d'arrêt (48).
